(19) Europäisches Patentamt
European Patent Office
Office européen des brevets

(11) Publication number: **0 326 320**
**A1**

(12) # EUROPEAN PATENT APPLICATION

(21) Application number: **89300637.9**

(51) Int. Cl.[4]: **C 07 C 147/10**

(22) Date of filing: **24.01.89**

(30) Priority: **25.01.88 GB 8801611**

(43) Date of publication of application:
**02.08.89 Bulletin 89/31**

(84) Designated Contracting States:
**AT BE CH DE ES FR GB IT LI LU NL SE**

(71) Applicant: **IMPERIAL CHEMICAL INDUSTRIES PLC**
**Imperial Chemical House Millbank**
**London SW1P 3JF (GB)**

(72) Inventor: **Walsingham, Richard Warren**
**18 Underwood**
**Kilwinning Ayrshire, KA13 7HR (GB)**

(74) Representative: **Chapman, Kenneth Hazel et al**
**Imperial Chemical Industries PLC Legal Department: Patents P.O. Box 6 Bessemer Road**
**Welwyn Garden City Herts, AL71HD (GB)**

(54) **Preparation of 4,4'-dihydroxydiphenyl sulphone.**

(57) 4,4′-dihydroxydiphenylsulphone is produced from a mixture containing also 2,4′-dihydroxydiphenylsulphone by

(a) heating the mixture in a solvent and isomerising 2,4′-dihydroxydiphenylsulphone to 4,4′-dihdroxydiphenylsulphone by removal of the solvent to yield crude 4,4′-dihydroxydiphenylsulphone;

(b) dissolving the crude sulphone in aqueous alkali;

(c) adding further alkali metal ions to precipitate 4,4′-dihydroxydiphenylsulphone as a mono alkali salt; and

(d) separating that salt.

The mono alkali salt may be dissolved in water and precipitated by acid, to give the sulphone in free acid form. The sulphone is of high purity and may with advantage be used in making polymers especially polyarylethersulphones. The mono alkali salt is believed to be a new compound.

EP 0 326 320 A1

## Description

### Preparation of 4,4′-dihydroxydiphenyl sulphone

This invention relates to the preparation of 4,4′-dihydroxydiphenyl sulphone, commonly referred to as "Bis-S".

Bis-S, is used in preparation of engineering polymers including polyethersulphones, polyesters, polycarbonates and epoxy resins, must be highly pure, otherwise there may result a polymer with inferior mechanical, electrical or optical properties. Moreover it is difficult to wash potassium salts from polyethersulphones made from impure Bis-S.

Percentages referred to in this specification are by weight unless otherwise indicated.

A conventional process for manufacture of Bis-S, disclosed in US 4162270 involves reaction of phenol with sulphuric acid to give a mixture of 2,4′- and 4,4′-dihydroxydiphenyl sulphones and subsequent isomerisation of the 2,4′-insomer to the 4,4′-isomer in the presence of an halogenated solvent such as 1,2-dichlorobenzene at 180°. The solvent not only allows azeotropic removal of water but selectively dissolves the 2,4′-dihydroxydiphenyl sulphone, allowing isomerisation of the latter to the 4,4′-insomer. The solvent is gradually removed from the reaction mixture, resulting in a high yield of comparatively pure Bis-S. However further purification is necessary. Treatment of a solution of Bis-S in sodium hydroxide with carbon, filtration and precipitation with sulphuric acid has been employed. Two cycles of treatment with carbon resulted in a yield of 76% of a product which contains 0.18% of the 2,4′-isomer and less than 0.05% of tris-phenol disulphone by-products. The melting point of 247.8° indicated that the product is insufficiently pure for high quality applications.

A higher quality product has been obtained by washing the crude product with aqueous sodium hydroxide prior to the treatment with carbon but a lower yield is obtained eg 54%.

According to the invention a process for preparation of a salt of 4,4′-dihydroxydiphenyl sulphone from a mixture containing also 2,4′- dihydroxydiphenyl sulphone comprises:

(a) heating the mixture in a solvent and isomerising 2,4′-dihydroxydiphenyl sulphone to 4,4′-dihydroxydiphenyl sulphone by removal of the solvent to yield crude 4,4′-dihydroxydiphenyl sulphone,

(b) dissolving the crude sulphone in aqueous alkali,

(c) adding further alkali metal ions to precipitate 4,4′-dihydroxydiphenyl sulphone as a mono alkali salt: and

(d) separating that salt,

The process may further comprise:

(e) dissolving the mono alkali salt in water; and

(f) adding acid to precipitate purified 4,4′-dihydroxydiphenyl sulphone.

The product from removal of the solvent is referred to hereafter as "crude sulphone".

The invention provides a process for producing a polymer by reacting 4,4′-dihdroxydiphenylsulphone produced by the process of the invention with an appropriate difunctional reactant such as a dibasic carboxylic acid or ester-forming derivative thereof, or an ester-forming derivative of carbonic acid or a dihalogenoaromatic compound. The reaction with a dihalogenoaromatic compound, giving a polyarylethersulphone, is especially advantageous in virtue of the high quality of the product.

The dihalogenoaromatic compound preferably contains at least one electron - withdrawing group positioned so as to activate the halogens. Examples are 1,3-dihalo-2-cyanobenzene and compounds of formula

$X(Ar - Y)_n - Ar - X$ where X is halogen Y is $SO_2$ or CO, Ar is paraphenylene or 4,4′-biphenyl and n is 1 or 2. Particular examples are

4.4′-dichlorodiphenylsulphone

4.4′-difluorodiphenylsulphone

4.4′-difluorobenzophenone

4.4′-bis(4-chlorophenylsulphonyl) biphenyl

1.4 -bis(4-fluorobenzoyl) benzene

If the dihalogenoaromatic compound does not contain such an electron - withdrawing group or if the halogen is chlorine and more rapid reaction is needed, the reaction may be carried out in presence of a copper - containing catalyst.

The dihydric phenol component can comprise, in addition to the 4,4′-dihydroxydiphenylsulphone, one or more other dihydric phenols, for example one or more of

hydroquinone

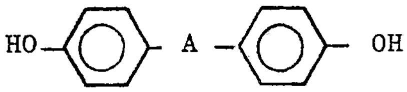 where A is CO, a direct link

Preferably a solvent is present, suitably of the dipolar aprotic type such as a sulphoxide or sulphone, and preferably of the formula

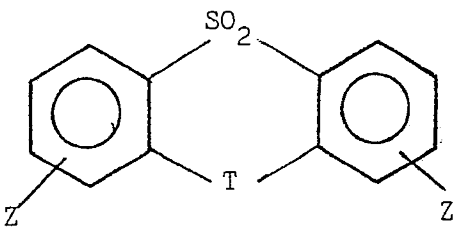

where T is a direct link or two hydrogen atoms (one attached to each benzene ring) and Z and Z′ are hydrogen or phenyl.

The reaction temperature is typically in the range 150-400 especially 200-400°C. Reaction takes typically 2-40 hours and is continued until a polymer of the required molecular weight, suitably in the range 2000 - 10000, is formed.

The reaction is carried out in presence of a base in quantity at least sufficient (preferably with 1-25% excess) to neutralise the hydrogen halide notionally produced by the reaction. The base is suitably one or more alkali metal compounds, which can be introduced by initially forming a salt of the phenolic component but preferably by having their carbonates present. The metal of the base preferably is potassium or contains enough potassium along with sodium to give an atomic ratio K/(K+Na) in the range 0.1 to 10.0%. Other alkali metals and/or alkaline earth metals can be present. Provided sufficient base-equivalent is present, part of the alkali can be introduced as neutral salts, eg halide, especially fluoride.

The monoalkali salt can be used in polymer production without further purification, apart from possibly drying it. Thus the invention provides a process for producing a polyarylethersulphone by bringing together a monoalkali salt of 4,4′-dihydroxy diphenylsulphone, further alkali to increase the total alkali content to 100-125% equivalence to the total phenolic hydroxy groups present and a dihalogenoaromatic component and reacting these components with separation of alkali halide. The further alkali can be a hydroxide and thus capable of forming a bis-alkali metal salt with the unneutralised OH of the sulphone and any other phenols that may be present. More preferably it is a carbonate or bicarbonate, so as to limit the concentration of phenate present, if desired, the monoalkali salt can be treated with $CO_2$.

In step (a) the solvent is suitably an aromatic hydrocarbon or chlorinated hydrocarbon having a boiling point in the range 100-220°C. The isomerisation temperature is suitably in the range 100-200°C, especially over 160°C. As isomerisation proceeds, the 4,4′-sulphone crystallises out.

In step (c) the metal of the salt is preferably sodium owing to the low water solubility of the sodium salt but can be another alkali metal, especially potassium.

Substantially all the 4,4′-sulphone is preferably separated as the mono alkali salt. When the alkali metal ions are provided by adding alkali hydroxide, the amount of alkali may be varied. With less added alkali a proportion of sulphone in acid form is also separated. With more added alkali some product is lost at the separation stage as the soluble di-alkali salt. A neutral alkali metal salt for example sodium sulphate, may be added to reduce the solubility of the mono alkali salt without increasing alkalinity. The effect of adding alkali is illustrated particularly by Example 6 below. For precipitation of the mono alkali salt the pH is preferably in the range 1-3 units on the alkaline side of neutrality applicable at the temperature of measurement.

In an alternative process the crude sulphone may be dissolved in more than sufficient alkali to form the mono alkali salt, the mono alkali salt being precipitated by the addition of an acid, for example sulphuric acid. The neutral alkali metal salt is thus formed by reaction of the acid with the dialkali salt, and, as above, enhances the yield of the monosodium salt common ion effect.

When the mono sodium salt is to be separated, the sodium ion concentration is typically in the range 1-3 mol per 1000 g of solution.

Additional purification can be achieved by dissolving the separated mono alkali salt in sufficient alkali to produce a solution of the di-alkali salt and re-forming the mono-alkali salt by adding an appropriate quantity of acid.

The process of this invention is advantageous in that isolation of the mono-alkali salt at relatively high pH avoids contamination with soluable impurities which would co-precipitate from lower pH solutions. A high yield and throughput may be attained by operation of the process at high concentrations.

Dissolution of the sulphone and separation of mono alkali salt obviates one of the disadvantages of processes wherein the crude sulphone is washed with aqueous alkali because the latter processes are sensitive to the crystalline form of the crude material and the impurities therein, in particular to impurities which are occluded within the crystals. The yield was also sensitive to the quantity of aqueous alkali used for the washing.

The process may afford a yield of 68% of 4,4′-dihydroxydiphenyl sulphone having a melting point of 248.5° and containing not more than 0.06% of the 2,4′-isomer.

Any convenient alkali or mixture of alkalis may be used. Use of sodium hydroxide is economical although potassium hydroxide may also be employed.

Impure sulphone can be precipitated by addition of acid to the filtrates derived from separation of mono-alkali salt, then separated and recycled to step (a) for isomerisation together with the main streams of reactants and purification via the mono alkali salt. If the acid added to the filtrates is insufficient to effect total precipitation, then the precipitate is relatively rich in 4,4′-dihydroxydiphenyl sulphone and can be recycled to step (b) for recovery of that isomer as monoalkali salt. Recycling results in a substantial improvement in yield.

The concentration of crude sulphone in aqueous alkali for precipitation of mono alkali salt can be varied within the range 1 part crude sulphone to between 1 part and 5 parts by weight of the solution of alkali. However, it is only at the higher concentration that a yield of 68% can be achieved.

The temperature at which the mono alkali salt is precipitated by addition of acid or alkali is preferably in the range 40° to 100°C, more preferably 80° to 100°C because this reduces the proportion of 2,4′-isomer in the product.

The separation of mono alkali salt may be performed at temperature of 20° to 100°, preferably 30° to 60°C, more preferably at about 40°, at which temperature the yield and purity is optimised.

The alkali can be added in a number of ways, for example as a single charge into water prior to addition of the crude sulphone or (preferably) as a first charge sufficient to enable solution of the crude sulphone followed by a second charge that precipitates the mono alkali salt from solution. A temperature of at least 80° may be needed to achieve solution of the crude sulphone following the initial alkali charge when the concentration of 1 part crude sulphone to 1 part aqueous alkali is employed.

The most pure and preferred final product from step (f) may be obtained when the crude sulphone is fully dissolved prior to precipitation of mono alkali salt by adding the second charge of alkali.

Further purification may be achieved by dissolving the separated alkali salt in water, treating with active carbon and re-precipitating with acid. However because of the relatively high purity of the mono alkali salt, the quantity of water used for dissolution can be substantially less allowing processing at higher concentration than in US 4162270.

The monosodium and monopotassium salts of 4,4′-dihydroxy diphenylsulphone are believed to be new compounds.

This invention is further described by means of example, but not in any limitative sense.

Crude sulphone from isomerisation in step (a) of the mixture of 2,4′-and 4,4′-dihydroxydiphenyl sulphones in accordance with the process disclosed in US 4162270 comprised

| | |
|---|---|
| 4.4′-dihydroxydiphenyl sulphone | 83 - 87% |
| 2.4′-dihydroxydiphenyl sulphone | 1.5 - 2.5% |
| triphenyhldisulphones | 1.0 - 2.0% |
| sulphonic acids and other impurities | balance |
| | 100% |

The melting point of the crude product is 220° The molar quantities of sodium hydroxide used in the following examples are based upon the quantity of sulphuric acid used in the phenol sulphonation reaction from which the reaction mixture subjected to the isomerisation in step (a) was derived.

Example 1

Crude sulphone (100 g, 0.425 mol) was added to water (150 $cm^3$) and the slurry stirred at 90°. Aqueous sodium hydroxide (47%, 18.1 g, 0.5 mol/mol sulphuric acid) was added and the mixture stirred for 30 min whereupon the sulphone dissolved. Aqueous sodium hydroxide (47%, 21.7 g, 0.6 mol/mol sulphuric acid) was

added over 1 h, whereupon 4,4′-dihydroxydiphenyl sulphone monosodium salt crystallised from solution. The mixture was cooled to 40° over 3 h and filtered.

The resultant damp cake (97.9 g, 90% solids) was dissolved in water (700 $cm^3$) at 90°. Decolorising carbon (2.0 g) was added and the mixture was stirred at 90° for 1 h, then filtered to remove the carbon. The filter cake was washed with water (20 $cm^3$) and the washings combined with the batch solution. The whole solution was stirred at 90° and ethylene diamine tetra acetic acid (0.36 g) and sodium hydrosulphite (0.2 g) were added. Sulphuric acid (77%, 27g) was added over 1 h and the pH adjusted to 6.0 at 90° whereupon a white precipitate formed. The resulting suspension was heated to 95° and allowed to cool to 40° (3h), the pH rising to 7.0. The suspension was allowed to stand for 1 h, small amounts of sodium hydrosulphite being added to remove any coloration which developed. The suspension was filtered and the filter cake washed with water (100 $cm^3$). The product was analysed as follows, the yield being calculated from the quantity of sulphuric acid used in the sulphonation reaction.

| Material | Dry weight (g) | 4,4′-Isomer Yield (%) | % Impurities 2,4′ | Tris | mp (°C) |
|---|---|---|---|---|---|
| Crude sulphone | 100 | 80 | 2.0 | 1.5 | 220 |
| Mono Sodium salt | 88.1 | 72 | 0.35 | 0.05 | > 300 |
| Bis S | 72.2 | 68 | 0.07 | 0 | 248.4 |

In variants of Example 1, (a) the separated salt was washed with water prior to further processing (b) the volume of water for dissolving sodium salt was decreased to 550 $cm^3$ and (c) the volume of water used for dissolving the sodium salt was decreased to 400 $cm^3$. The products arising from these variants were analysed as follows:

| Material | Variant | Dry Weight (g) | 4,4′-Isomer Yield (%) | % Impurities 2,4′ | Tris | mp (°C) |
|---|---|---|---|---|---|---|
| Crude sulphone | | 100 | 80 | 2.0 | 1.5 | 220 |
| Mono Sodium Salt | (a) | 85.7 | 70 | | | > 300 |
| Bis-S | (a) | 70.1 | 66 | 0.06 | 0 | 248.5 |
| Bis-S | (b) | 72.7 | 68.5 | 0.09 | 0 | 248.3 |
| Bis-S | (c) | 73.3 | 69.0 | 0.11 | 0 | 248.0 |

The identity of the monosodium salt was established by analysis of its sodium content.

Example 2

The procedure of Example 1 was followed but the quantity of 47% aqueous sodium hydroxide added over 1.0 h to the solution of crude sulphone was in variant (a) decreased to 7.2 g (0.2 mol/mol) and in variant (b) increased to 32.6 g (0.9 mol/mol).

The products arising from these variants were analysed as follows:

| Material | Variant | Dry Weight (g) | 4,4′-Isomer Yield (%) | % Impurities 2,4′ | Tris | mp (°C) |
|---|---|---|---|---|---|---|
| Crude sulphone | | 100 | 80 | 2.0 | 1.5 | 220 |
| Mono Sodium Salt | (a) | 89.0 | 72 | 1.2 | 0.3 | > 300 |
| Bis-S | (a) | 72.3 | 68 | 0.23 | 0 | 247.9 |
| Mono Sodium Salt | (b) | 62.4 | 51 | 0.35 | 0.05 | > 300 |
| Bis-S | (b) | 51.0 | 48 | 0.07 | 0 | 248.3 |

Example 3

The procedure of Example 1 was followed but the slurry of crude sulphone was held at 60°C in variant (a) and at 40°C in variant (b) for the additions of aqueous sodium hydroxide. In both of these variants there was solid material present throughout the aqueous sodium hydroxide addition.

The product arising from these variants was analysed as follows:

| Material | Variant | Dry weight (g) | 4,4'-Isomer Yield (%) | % Impurities 2,4' | Tris | mpt (°C) |
|---|---|---|---|---|---|---|
| Crude sulphone | | 100 | 80 | 2.0 | 1.5 | 220 |
| Mono Sodium salt | (a) | 89.0 | 72 | 1.2 | 0.2 | |
| Mono Sodium salt | (b) | 89.5 | 72 | 1.4 | 0.2 | |

Example 4

Crude sulphone (100 g, 0.425 mol) was added to a solution of water (150 $cm^3$) and aqueous sodium hydroxide (47%, 72.3 g, 2.0 mol/mol) and the slurry was stirred at 90°C whereupon all solid material dissolved. Sulphuric acid (77%, 24.3 g, o.45 mol/mol) was added over 1 h, whereupon 4,4'-dihydroxydiphenyl sulphone monosodium salt crystallised from solution. The mixture was cooled to 40° over 3h and filtered.

The resultant damp cake (128.8 g, 80% solids) was dissolved in water (700 $cm^3$) at 90°C and thereafter treated according to the procedure of Example 1. The product was analysed as follows:

| Material | Dry weight (g) | 4,4'-Isomer Yield (%) | % Impurities 2,4' | Tris | mp (°C) |
|---|---|---|---|---|---|
| Crude sulphone | 100 | 80 | 2.0 | 1.5 | 220 |
| Mono Sodium salt | 103 | 78 | 0.56 | 0.06 | > 300 |
| Bis-S | 79.7 | 75 | 0.15 | ND | 248.3 |

Example 5

Crude sulphone (100 g, 0.425 mol) was added to water (250 $cm^3$) and the slurry stirred at 90°C. Aqueous sodium hydroxide (47%, 18.1 g, 0.5 mol/mol sulphuric acid) was added and the mixture stirred for 30 min whereupon the sulphone dissolved. Aqueous sodium hydroxide (47% 21.7 g, 0.6 mol/mol) was added over 1 h, whereupon 4,4'-dihydroxydiphenyl sulphone monosodium salt crystallised from solution. The mixture was cooled to 40°C over 3 h and filtered.

The resulting damp cake (79.8 g, 90% solids) was treated according to the procedure of Example 1 to produce 59.2 g dry product.

The temperature of the filtrates from separation of the monosodium salt (270.2 g) was adjusted to 40° and sulphuric acid (77%, 12.6 g) added over 1 h to adjust the pH to 7.5. Impure bis phenol sulphone crystallised as a white solid and was separated by filtration.

The recovered white solid (41.4 g, 70% solids) was added with crude sulphon (100 g, 0.425 mol) into water (250 $cm^3$) and the slurry processed by the procedure of Example 1 with corresponding adjustment of reactant charges to account for increased molar quantity of material present.

This procedure provided 4,4'-dihydroxydiphenyl sulphone monosodium salt damp cake (123.3 g, 90% solids) and finally 80.0 g of final Bis-S product. Filtrates remaining after separation of the sodium salt were also available for recovery of impure bis phenol sulphone which could be further recycled. The products of this Example were analysed as follows :

| Material | Dry Weight (g) | 4,4'-Isomer Yield (%) | % Impurities 2,4' | Tris | mp (°C) |
|---|---|---|---|---|---|
| Crude sulphone | 100 | 80 | 2.0 | 1.5 | 220 |
| Mono Sodium salt | 71.8 | 58 | 0.35 | 0 | |
| Bis-S | 59.2 | 55.7 | 0.06 | 0 | 248.4 |
| Impure Bis-S | 29.0 | 24 | 1.9 | 0.34 | |
| Mono Sodium salt (2nd stge) | 111.0 | 78.0* | 0.23 | 0 | |
| Bis-S (2nd stage) | 80.0 | 75.3* | 0.04 | 0 | 248.5 |

* The yield of 4,4'-isomer is calculated based on the charge of crude sulphone processed in the second stage.

Example 6

When aqueous sodium hydroxide (47%) was added to a slurry of crude sulphon (100 g, 0.425 mol) in water (150 $cm^3$) stirred at 90°C, the appearance and the pH of the mixture followed a characteristic pattern. There was an initial sharp increase in pH from 1.5 to 7.0 following the addition of 0.1 mol/mol aqueous sodium hydroxide and a colour change from pink to buff. Thereafter the pH increased more gradually to 8.0 after 1.0 mol/mol aqueous sodium hydroxide was added. During this phase of the addition the crude sulphone was dissolved to give complete solution, and 4'4,-dihydroxydiphenyl sulphone monosodium salt started to crystallise at the 0.5 mol/mol and 0.7 mol/mol addition stages respectively.

Further addition of aqueous sodium hydroxide (47%) up to 1.9 mol/mol caused the pH to gradually increase up to 10 as the monosodium salt was gradually dissolved to become fully in solution. Addition of aqueous sodium hydroxide (47%) up to the 2.0 mol/mol level then caused the pH to increase more steeply to 11.

(These pH values were measured at 90°C and are to be compared with the value for neutrality at that temperature and at the salt concentration present in the mixture.

## Claims

1. A process for preparation of a salt of 4,4'-dihydroxydiphenylsulphone from a mixture containing also 2,4'-dihydroxydiphenylsulphone which comprises
   (a) heating the mixture in a solvent and isomerising 2,4'-dihydroxydiphenylsulphone to 4,4'-dihydroxydiphenylsulphone by removal of the solvent to yield crude 4,4'-dihydroxydiphenylsulphone; (b) dissolving the crude sulphone in aqueous alkali; (c) adding further alkali metal ions to precipitate 4,4'-dihydroxydiphenylsulphone as a mono alkali salt; and (d) separating that salt.
2. A process for producing purified 4,4'-dihydroxydiphenyl sulphone by making its mono alkali salt by the process of claim 1, then
   (e) dissolving the mono alkali salt in water; and (f) adding acid to precipitate purified 4,4'-dihdroxydiphenylsulphone.
3. A process according to claim 1 or claim 2 in which in step (c) the further alkali metal ions are added as alkali hydroxide.
4. A process according to any one of claims 1 to 3 in which in step (c) further alkali metal ions are added as neutral alkali metal salt or by dissolving the sulphone in more than sufficient alkali to form the monoalkali salt and then adding acid.
5. A process according to any one of the preceding claims in which the mono alkali salt is separated from solution at a pH 1 to 3 units on the alkaline side of neturality.
6. A process according to any one of the preceding claims in which step (c) is carried out at a temperature in the range 80-100°C and step (d) is carried out at a temperature in the range 30-60°C.
7. A process according to any one of the preceding claims in which impure sulphone is precipitated by addition of acid to the filtrates derived from separation of mono alkali salt and is recycled to step (a) for isomerisation or (if acid addition is insufficient to effect total precipitation) to step (8).
8. The monosodium or monopotassium salt of 4,4'-dihdroxydiphenylsulphone.
9. A process for producing a polyarylethersulphone by reacting a dihalogenoaromatic compound with a dihydric phenol component comprising 4,4'-dihydroxydiphenylsulphone, characterised in that the 4,4'-dihydroxydiphenylsulphone is the product of a process according to any one of claims 1 to 7.

10. A process for producing a polyarylethersulphone by bringing together a dihydric phenol component comprising a monoalkali salt of 4,4′-dihydroxydiphenylsulphone, further alkali to increase the total alkali content to 100-125% equivalence to the total phenolic hydroxy groups present and a dihalogenoaromatic compound component and reacting these components with separation of alkali halide.

European Patent Office

# EUROPEAN SEARCH REPORT

Application Number

EP 89 30 0637

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | LIEBIGS ANNALEN DER CHEMIE, vol. 147, 1868, pages 52-66, Berlin, DE; L. GLUTZ: "Untersuchungen aus dem chemischen Laboratorium des Professor Kolbe. LVII. Ueber Oxysulfobenzid und einige Derivate desselben"<br>* Page 56 *<br>--- | 1,8 | C 07 C 147/10 |
| L | BEILSTEINS HANDBUCH DER ORGANISCHEN CHEMIE, 4th edition, vol. 6, 1923, page 861, Verlag von Julius Springer, Berlin, BE; "Isocyclische oxy-Verbindungen"<br>* Page 861, paragraph 3, lines 1,23, gives up to date molecular formula and name for compound cited in above X document *<br>--- | | |
| A | US-A-2 392 137 (S.H. FOSTER)<br>* Page 3, column 1 - page 4, column 2; claims 1,3-7 *<br>--- | 1-8 | TECHNICAL FIELDS SEARCHED (Int. Cl.4) |
| A | EP-A-0 220 855 (AMOCO CORP.)<br>* Claims *<br>--- | 1-8 | C 07 C 147/00 |
| D,A | US-A-4 162 270 (E. OGATA et al.)<br>* Column 4, lines 59-68 *<br>----- | 1-8 | |

The present search report has been drawn up for all claims

| Place of search | Date of completion of the search | Examiner |
|---|---|---|
| THE HAGUE | 10-04-1989 | ZAROKOSTAS K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document